# EUROPEAN PATENT APPLICATION

(11) **EP 3 824 814 A1**
(43) Date of publication of application: **26.05.2021**
(21) Application number: 19210887.6
(22) Date of filing: 22.11.2019
(51) Int. Cl.: A61B 6/03, A61B 6/00, G01R 33/56, G01R 33/58

(54) **ASSESSMENT OF MEASURED TOMOGRAPHIC DATA**

(71) Applicant: Koninklijke Philips N.V., 5656 AG Eindhoven (NL)
(72) Inventor: STASSEN, Maurice Leonardus Anna, 5656 AE Eindhoven (NL); NETSCH, Thomas, 5656 AE Eindhoven (NL)
(74) Representative: Philips Intellectual Property & Standards

(57) **Abstract**

Disclosed herein is a medical instrument (100, 300, 400, 500) comprising: a memory (110) storing machine executable instructions (120) and a tomographic data assessment module (122) and a processor (106) configured for controlling the medical instrument. Execution of the machine executable instructions causes the processor to receive (200) measured tomographic data (124). The measured tomographic data is configured for being reconstructed into a tomographic image (308) of a subject (418). Execution of the machine executable instructions further causes the processor to receive (202) an image quality indicator (126, 126', 126") by inputting the measured tomographic data into the tomographic data assessment module. The tomographic data assessment module is configured for generating the image quality indicator in response to inputting the measured tomographic data. Execution of the machine executable instructions further causes the processor to provide (204) the image quality indicator to an operator using an operator signaling system (108).

## Description

### TECHNICAL FIELD

The invention relates to tomographic imaging methods, in particular to magnetic resonance imaging and computed tomography

### BACKGROUND OF THE INVENTION

In tomographic imaging methods data is acquired from a subject and are reconstructed into tomographic images. Tomographic images such as magnetic resonance imaging or computed tomography involve the use of expensive medical imaging systems.

International patent application WO 2018/220089 A1 discloses a raw diagnostic machine for a medical diagnosis of raw medical imaging data generated by a medical imaging machine as opposed to a medical diagnosis of a medical image conventionally reconstructed from the raw medical imaging data. In operation, the raw diagnostic engine includes a medical imaging diagnostic controller implementing a dimension reduction pre-processor for selecting or extracting one or more dimension reduced feature vectors from the raw medical imaging data, and further implementing a raw diagnostic artificial intelligence engine for rendering a diagnostic assessment of the raw medical imaging data as represented by the dimension reduced feature vector(s). The medical imaging diagnostic controller may further control a communication of the diagnostic assessment of the raw medical imaging data (e.g., a display, a printing, an emailing, a texting, etc.).

### SUMMARY OF THE INVENTION

The invention provides for a medical instrument, a method and a computer program product in the independent claims. Embodiments are given in the dependent claims.

As mentioned above tomographic imaging techniques involve expensive medical imaging systems to acquire measured tomographic data. When a subject is imaged in a medical imaging system the operator of the medical imaging system waits until the tomographic image has been reconstructed from the measured tomographic data. The operator then examines the tomographic image and decides if the image is of sufficient quality or not. The operator then decides to discharge the subject or to reacquire the measured tomographic data. This introduces a delay for the subject and reduces the number of subjects which can be imaged by the medical imaging system.

Embodiments may provide for means saving time by inputting the measured tomographic data into a tomographic data assessment module before reconstruction of the tomographic image. In response the measured tomographic data assessment module provides an image quality indicator which may for example be used to predict the quality of the tomographic image if it is reconstructed. The operator can for example make the decision to discharge the subject or to reacquire the measured tomographic data on the basis of the image quality indicator. If the subject is discharged the tomographic image may be reconstructed from the measured tomographic data at a later time.

In one aspect the invention provides for a medical instrument that comprises a memory storing machine-executable instructions and a tomographic data assessment module. The medical instrument further comprises a processor that is configured for controlling the medical instrument. Execution of the machine-executable instructions causes the processor to receive measured tomographic data. The measured tomographic data is configured for being reconstructed into a tomographic image of a subject. Execution of the machine-executable instructions further causes the processor to receive an image quality indicator in response to inputting the measured tomographic data into the tomographic data assessment module. The tomographic data assessment module is configured for generating an image quality indicator in response to inputting the measured tomographic data.

The measured tomographic data used herein encompasses the measured data from a medical imaging system. For example, the measured tomographic data may include measured data from a magnetic resonance imaging system and a computed tomography system. Execution of the machine-executable instructions further causes the processor to provide the image quality indicator to an operator using an operator signaling system. In this embodiment the image quality indicator operates in some examples directly on the measured tomographic data.

This embodiment may be beneficial because it may provide for a means of assessing the quality of the tomographic image before it has been reconstructed. For example, if there is a lengthy and complicated reconstruction algorithm for reconstructing the tomographic image from the measured tomographic data it may consume a large amount of time. The image quality indicator may therefore be used to decide to reacquire the measured tomographic data without a delay in the reconstruction of the tomographic image.

In another embodiment the medical instrument further comprises a medical imaging system configured for acquiring the measured tomographic data from an imaging zone. The memory further comprises medical imaging system control commands configured for controlling the medical imaging system to acquire the measured tomographic data.

For example, the medical imaging system may comprise various components which need to be operated in a coordinated fashion as a function of time. The medical imaging system control commands may be used to control these various components to acquire the measured tomographic data. A concrete example is the pulse sequence or pulse sequence commands used by a magnetic resonance imaging system and various components and amplifiers are controlled in concert to acquire the measured tomographic data. Execution of the machine-executable instructions further causes the processor to acquire the measured tomographic data by controlling the medical imaging system with the medical imaging system control commands.

In another embodiment the medical imaging system further comprises a subject support for moving at least a portion of the subject within the imaging zone. Execution of the machine-executable instructions further causes the processor to control the subject support to move the at least a portion of the subject within the imaging zone before controlling the medical imaging system to acquire the measured tomographic data. Execution of the machine-executable instructions further causes the processor to provide the image quality indicator to the operator using the operator signaling system while the subject is still supported at least partially within the imaging zone. This embodiment may be beneficial because when the subject is still within the imaging zone the measured tomographic data may be reacquired. The use of the tomographic data assessment module enables the quality of the measured tomographic data to be assessed while the subject is still within the medical imaging system. This provides an opportunity to reacquire data if necessary.

In another embodiment the medical imaging system is a magnetic resonance imaging system. The medical imaging system control commands are pulse sequence commands. The measured tomographic data is k-space data. Magnetic resonance imaging systems acquire data in k-space which is then Fourier transformed into the final tomographic image. In this example the tomographic image is a magnetic resonance image. The reconstruction algorithms for reconstructing a magnetic resonance image may be very time and computationally intensive. This embodiment may be beneficial because it provides the opportunity to assess whether the measured tomographic data will likely result in a quality tomographic image or not. This enables subjects to be discharged before the tomographic image has been reconstructed. This may result in significant savings in the amount of time that each subject uses for the magnetic resonance imaging system.

In another embodiment the pulse sequence commands are according to a compressed sensing magnetic resonance imaging protocol configured for acquiring the measured tomographic data from multiple magnetic resonance imaging antennas. The tomographic data assessment module is configured for at least partially providing the image quality indicator using magnetic resonance data from a single magnetic resonance antenna selected from the multiple magnetic resonance imaging antennas. In compressed sensing data is acquired from multiple antenna or antenna elements and each of these is used to reconstruct an image. Coil sensitivity profiles are then used to combine these images into a single magnetic resonance image or images. By using the data from a single antenna this may reduce the amount of time necessary to reconstruct a trial image or to examine the data. This may result in a large acceleration of the time used to generate the image quality indicator.

In another embodiment the pulse sequence commands are according to a self-navigating magnetic resonance imaging protocol that embeds self-navigator data within the k-space data. Often times the central region of k-space can be repeatedly measured or oversampled so that the data can be used as a self-navigator. This embodiment may be beneficial because it may be useful in measuring the degree of motion of the subject.

In another embodiment the medical imaging system is a computed tomography imaging system. The measured tomographic data comprises measured X-ray attenuation profiles. This embodiment may also be beneficial because the reconstruction algorithms for computed tomography may also take a significant amount of time to produce the finished tomographic image. This may be particularly true in cases where the computed tomography system makes measurements at multiple X-ray tube voltages or X-ray energies.

In another embodiment the tomographic data assessment module is configured for accelerating the generation of the image quality indicator by subsampling the measured tomographic data. For example, the measured tomographic data may be a very large dataset that is acquired over a period time. The amount of data which is necessary to perform a reconstruction may be reduced by just taking a portion of this measured tomographic data. This may result in for example a lower quality or an image with lower contrast which contains less information but may still be useful in assessing the overall quality of the measured tomographic data.

The tomographic data assessment module is configured for accelerating the generation of an image quality indicator by reconstructing a low-resolution image from the measured tomographic data. The low-resolution image has a lower resolution than the tomographic image.

In another embodiment the generation of the image quality indicator is accelerated by reconstructing a single slice of the tomographic image from the measured tomographic data. This may for example be useful in reducing the amount of data necessary to obtain the image quality indicator.

In another embodiment the measured tomographic data comprises redundant data. The tomographic data assessment module is configured to at least partially generate the image quality indicator using the redundant data. For example, the medical imaging system could be configured to make identical measurements several times during the course of acquiring the measured tomographic data. These measurements could then be compared to each other to note such things as degradation in image quality or movement of the subject.

In another embodiment the tomographic data assessment module is implemented as a neural network trained to receive as input the measured tomographic data and in response output the image quality indicator. In this embodiment the neural network essentially receives the raw (measured tomographic data) data from the medical imaging system and then outputs the image quality indicator.

In another embodiment the tomographic data assessment module is implemented as a logic module configured to receive as input the measured tomographic data and in response output the image quality indicator. The predetermined logic module may for example be a rule-based module that when certain conditions are indicated the image quality indicator is assigned.

In another embodiment the tomographic data assessment module is implemented as an operator-controlled module configured to generate and display intermediate images for approval by the operator. For example, the operator-controlled module may display a dialogue box on a graphical user interface which displays the image quality indicator or values on the display. The operator may then for example click whether the data or image is sufficient or acceptable.

In another embodiment execution of the machine-executable instructions further causes the processor to store the measured tomographic data in a tomographic data database system of a remote processing system if the image quality indicator meets a predetermined criterion. This predetermined criterion may be used to indicate that the measured tomographic data is acceptable. The remote processing system is configured to retrieve the measured tomographic data from the tomographic data database and then to reconstruct the tomographic image from the measured tomographic data. In this embodiment after the measured tomographic data has been stored a different system then reconstructs the tomographic image. This for example may enable a subject to be discharged immediately and then the tomographic image to be reconstructed at a later date or time.

In another embodiment the remote processing system is implemented at a separate location from the medical instrument. For example, the remote processing system may also be implemented as a cloud or virtual system.

In another embodiment the operator signaling system further comprises a computer display configured for displaying the image quality indicator. Execution of the machine-executable instructions further causes the processor to perform the action of displaying a reacquired data message to the operator if the image quality indicator does not meet the predetermined criterion. For example, the image quality indicator may be assigned a binary value of an insufficient image quality classification. In this case the message is then relayed to the operator to reacquire the measured tomographic data.

In another embodiment execution of the machine-executable instructions further causes the processor to display a discharge subject message to the operator if the image quality indicator meets the predetermined criteria. For example, if the image quality indicator is a binary value which indicates that the measured tomographic data meets or has a sufficient image quality indicator then the subject may be discharged. The discharge subject message may for example instruct the operator to remove the subject from a medical imaging system.

In another embodiment the memory further comprises an instruction database comprising operator instructions that describe how to improve the measured tomographic data quality. Execution of the machine-executable instructions further cause the processor to retrieve the operator instructions from the instruction database if the reacquire data message is displayed. Execution of the machine-executable instructions further causes the processor to display the operator instructions on the display if the reacquire data message is displayed on the display.

In another embodiment the image quality indicator is a binary indicator which indicates a sufficient image quality and an insufficient image quality. Actions by the medical may easily be assigned on the basis of this binary value. A predetermined criterion for evaluating the binary indicator would be a chosen state of the binary indicator.

In another embodiment the image quality indicator is a numerical indicator. For example, a numerical value may be assigned to the image quality indicator. A predetermined criterion for evaluating the numerical indicator would be a chosen value to use as a threshold.

In another embodiment the image quality indicator is a lower contrast and/or lower resolution image than the tomographic image. The image quality indicator may be used by a machine algorithm or a human to evaluate the quality of the measured tomographic data. A trained neural network or other machine learning algorithm could for example be used to evaluate the image quality indicator and compared to a predetermined criterion.

In another embodiment the image quality indicator comprises an operator provided assessment. For example, a box may be displayed on a user interface which is then checked or evaluated by the operator. The binary indicator, the image quality indicator, and/or the numerical indicator could be displayed for evaluation by an operator.

In another aspect the invention provides for a method of operating a medical instrument. The method comprises receiving measured tomographic data. The measured tomographic data is configured for being reconstructed into a tomographic image of a subject. The method further comprises receiving an image quality indicator by inputting the measured tomographic data into a tomographic data assessment module. The tomographic data assessment module is configured for generating an image quality indicator in response to inputting the measured tomographic data. The method further comprises providing the image quality indicator to an operator using an operator signaling system.

In another aspect the invention provides for a computer program product comprising machine-executable instructions and a tomographic data assessment module. Execution of the machine-executable instructions causes the processor to receive measured tomographic data. The measured tomographic data is configured for being reconstructed into a tomographic image of a subject. The machine-executable instructions further cause the processor to receive an image quality indicator by inputting the measured tomographic data into the tomographic data assessment module. The tomographic data assessment module is configured for generating an image quality indicator in response to inputting the measured tomographic data. Execution of the machine-executable instructions further causes the processor to provide the image quality indicator to an operator using an operator signaling system.

It is understood that one or more of the aforementioned embodiments of the invention may be combined as long as the combined embodiments are not mutually exclusive.

For example, the following embodiments described as clauses and labeled by letters may be freely combined singly or in multiple combinations to construct dependent claim features:
A. wherein the pulse sequence commands are according to a compressed sensing magnetic resonance imaging protocol configured for acquiring the measured tomographic data from multiple magnetic resonance imaging antennas, wherein the tomographic data assessment module is configured for at least partially providing the image quality indicator using magnetic resonance data from a single magnetic resonance antenna selected from the multiple magnetic resonance imaging antennas;
B. wherein the pulse sequence commands are according to a self-navigating magnetic resonance imaging protocol that embeds self-navigator data within the k-space data, wherein the tomographic data assessment module is configured for at least partially providing the image quality indicator using the self-navigator data;
C. wherein the tomographic data assessment module is configured for accelerating the generation of the image quality indicator by subsampling the measured tomographic data;
D. wherein the tomographic data assessment module is configured for accelerating the generation of the image quality indicator by reconstructing a low-resolution image from the measured tomographic data, wherein the low-resolution image has a lower resolution than the tomographic image;
E. wherein the tomographic data assessment module is configured for accelerating the generation of the image quality indicator by reconstructing a single slice of the tomographic image from the measured tomographic data;
F. wherein the tomographic data assessment module is implemented as a neural network trained to receive as input the measured tomographic data and in response output the image quality indicator;
G. wherein the tomographic data assessment module is implemented as a predetermined logic module configured to receive as input the measured tomographic data and in response output the image quality indicator;
H. wherein the tomographic data assessment module is implemented as an operator-controlled module configured to generated and display intermediate images or values for approval by the operator;
I. wherein the image quality indicator is a binary indicator indicating a sufficient image quality and an insufficient image quality;
J. wherein the image quality indicator is a numerical indicator;
K. wherein the image quality indicator is a lower contrast and/or lower resolution image than the tomographic image;
L. wherein the image quality indicator is an operator provided assessment; and
M. any combination including one or more of features A through L above.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiment's computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display. An operator signaling system may be a display.

Measured tomographic data as used here encompasses the measured signals recorded during a tomographic imaging technique. Examples include Magnetic Resonance imaging and Computed Tomography.

K-space data is defined herein as being the recorded measurements of radio frequency signals emitted by atomic spins using the antenna of a Magnetic resonance apparatus during a magnetic resonance imaging scan. k-space data is an example of measured tomographic data. A Magnetic Resonance Imaging (MRI) image or MR image is defined herein as being the reconstructed two-, three-, or four- dimensional visualization of anatomic data contained within the k-space data and is an example of a tomographic image. This visualization can be performed using a computer.

Likewise, X-ray attenuation profiles are defined herein as being the recorded measurements of X-ray attenuation measurements made during a computed tomography scan. X-ray attenuation profiles are example of measured tomographic data. A Computer Tomography (CT) image is defined herein as being the reconstructed two- or three-dimensional visualization of anatomic data from the X-ray attenuation profiles. This visualization can also be performed using a computer.

### BRIEF DESCRIPTION OF THE DRAWINGS

In the following preferred embodiments of the invention will be described, by way of example only, and with reference to the drawings in which:
Fig. 1 illustrates an example of a medical instrument;
Fig. 2 shows a flow chart which illustrates a method of operating the medical instrument of Fig. 1;
Fig. 3 illustrates a further example of a medical instrument;
Fig. 4 illustrates a further example of a medical instrument;
Fig. 5 illustrates a further example of a medical instrument; and
Fig. 6 shows a flow chart which illustrates a method of operating the medical instrument of Fig. 3, 4 or 5.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

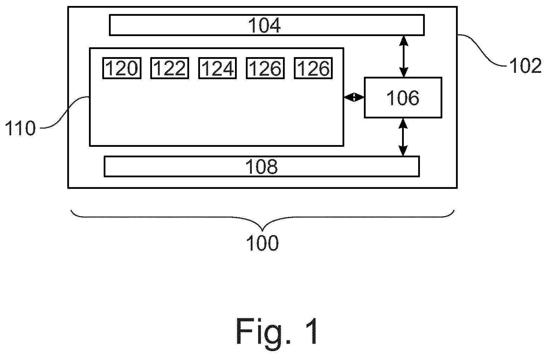

Fig. 1 illustrates an example of a medical instrument 100. The medical instrument is shown as comprising a computer 102 that has a processor 106 that is connected to a hardware interface 104, a user interface 108, and a memory 110. The hardware interface 104 may include components that enable the processor 106 to communicate or control them. The hardware interface 104 may also include one or more network interfaces. The processor 106 may be representative of one or more processors and/or processing cores. The user interface 108 may provide a means for an operator to interact with the computer 102. The user interface 108 may therefore provide a display or other operator signaling system. The memory 110 may be any combination of memory which is accessible for use by the processor 106.

The memory 110 is shown as containing machine-executable instructions 120. The machine-executable instructions 120 contain instructions which enable the processor 106 to control the function of the medical instrument 100 as well as provide various calculation and data processing tasks. The memory 110 is further shown as containing a tomographic data assessment module 122. The tomographic data assessment module receives raw tomographic data or measured tomographic data 124 as input and then outputs an image quality indicator 126. The memory 110 is shown as containing measured tomographic data 124 and then as output from the tomographic data assessment module 122 also contains the image quality indicator 126. The memory 110 is shown as further containing a rendering of the image quality indicator 128 which may be used or displayed using the user interface 108.

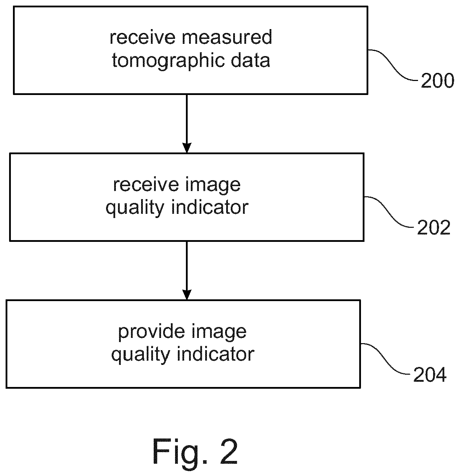

Fig. 2 shows a flowchart which illustrates a method of operating the medical instrument 100 of Fig. 1. First in step 200 the measured tomographic data 124 is received. Next in step 202 the measured tomographic data 124 is input into the tomographic data assessment module 122 and in response the image quality indicator 126 is received. Then finally in step 204, the image quality indicator 126 is provided to the operator. In this example the rendering of the image quality indicator 128 may be displayed using the user interface 108.

The current workflow of an MRI or CT scan consists of the following steps.
1. A patient is put in the scanner
2. A scan is made
3. The raw data from the scanner is reconstructed into an image
4. The operator performs a quality check, based on reconstructed image, if the scan was successful
5.
   a. If the scan was not successful a new scan needs to be made else the patient is dismissed
   b. If the scan was successful the reconstructed imaged is stored in the hospital's picture archiving and communication system (PACS)
6. A radiologist retrieves at a later stage the image from the PACS systems and writes a report of his findings/diagnosis. In an alternative the image from the PACS system can also be received in the scanner room, while the patient is still in the medical imaging system.

A disadvantage of the workflow described above is that the reconstruction process, step 3, can take quite some time. During this time the operator as well as the patient are waiting. This can be very inconvenient for the patient, since he/she is in an uncomfortable position and/or in a noisy and/or claustrophobic environment, particularly for MRI. The operator may feel he/she are wasting their time, while being under time pressure.

The computer for the above workflow should be as powerful as possible to keep the reconstruction time as short as possible.

Examples, may replace the current time-consuming reconstruction, step 3 in the workflow above, with an algorithm that determines based on the raw data (measured tomographic data) whether the scan has been successful or not. This algorithm may to require significantly less time than the actual reconstruction of a tomographic image from the measured tomographic data.

Based on the assessment of the algorithm (the image quality indicator), the patient can be dismissed. Therefore, the patient as well as the operator do not need to wait anymore.

The actual reconstruction of the tomographic image may be performed at a later moment, but in time for the radiologist to have the image ready when needed. The computer that does the reconstruction can now be less powerful since the reconstruction time is no longer a time critical step in the workflow.

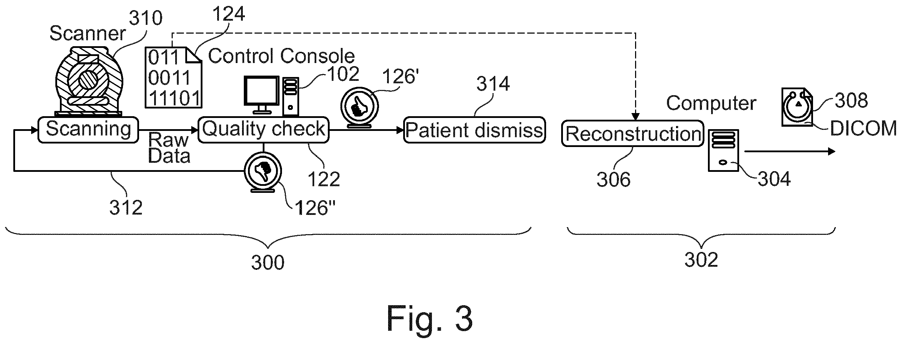

Fig. 3 illustrates a further example of a medical instrument 300. In addition, a remote processing system 302 is shown. In some cases, the remote processing system 302 can be part of the medical instrument 300. The medical instrument 300 is shown as comprising the computer 102. The items in Fig. 3 are arranged in a functional manner. The medical instrument 300 is further shown as comprising a medical imaging system 310 which is used to acquire the measured tomographic data 124. This is then input into the tomographic data assessment module 122. The tomographic assessment module 122 in this case is used to either provide a sufficient image quality indicator 126' or an insufficient image quality indicator 126".

If the sufficient image quality indicator 126' is provided then a message to dismiss the subject 314 is displayed. In addition, the raw data or the measured tomographic data 124 is transferred or transmitted to the remote processing system 302. The measured tomographic data 124 is then stored in a tomographic data database 306 for later processing. The remote processing system 302 comprises a computer 304. The computer is configured to retrieve the measured tomographic data 124 from the tomographic data database 306 and construct the tomographic image 308 from it. An advantage of this is that the subject may leave the medical imaging system 310 right away and the measured tomographic data 124 can be processed later, maybe even days later. This relieves the need to have more computing power in the computer 102 as well as enabling more patients per hour to go through the medical imaging system 310.

If the insufficient image quality indicator 126" is provided then the operator may be displayed a display reacquire data message 312. The operator can then reacquire the measured tomographic data 124. In some instances, the reacquisition of the measured tomographic data 124 may be automated.

The workflow with delayed reconstruction as illustrated in Fig. 3 may be summarized as:
1. A patient is put in the scanner
2. A scan is made
3. The raw data from the scanner is used for automatic quality check
4.
   a. If the scan was not successful a new scan needs to be made else the patient is dismissed
   b. If the scan was successful the raw data is stored
5. At a later moment the reconstruction of the tomographic image from the measured tomographic data is done and the reconstructed image (tomographic image) is stores in the hospital's picture archiving and communication system (PACS)
6. A radiologist retrieves at a later stage the image from the PACS systems and writes a report of his findings/diagnosis

An example of an algorithm for step 3 would to be to only reconstruct a single slice, without any advanced processing. Another option would be for the scanner to add (a small amount) of additional raw data which allows fast checks. Next to the scanner output, the algorithm could make use of additional sensors (cameras) mounted to the scanner. For example, if the patient has not moved during the scan, it is more likely the scan was successful.

All these options could be used to judge image quality with full reconstruction and to decide whether a rescan is needed or whether the patient can be dismissed.

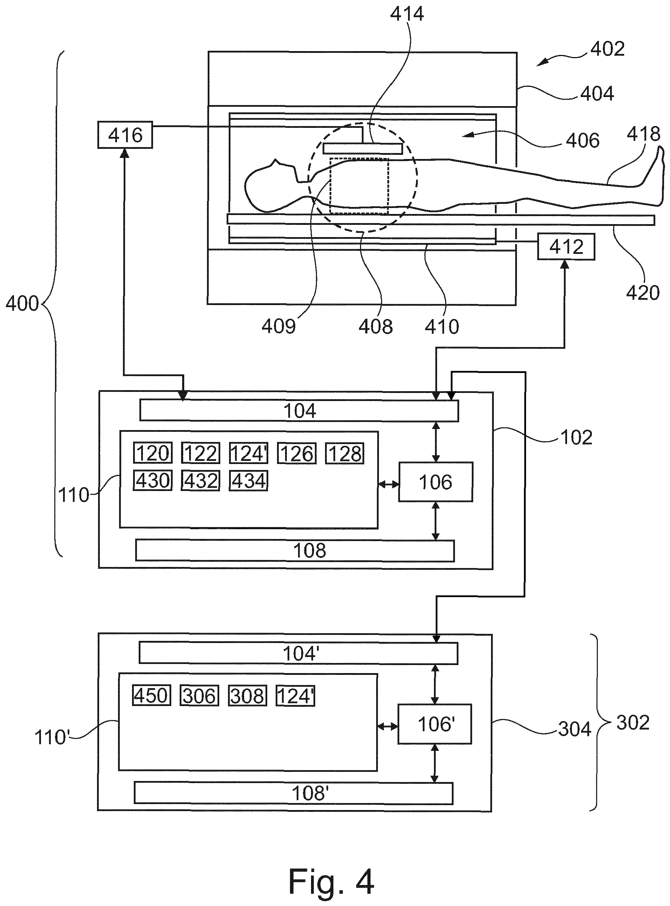

Fig. 4 illustrates a further example of a medical instrument 400. The example shown in Fig. 4 is similar to that illustrated in Figs. 1 and 3 except the medical instrument 400 additionally comprises a magnetic resonance imaging system 402.

The magnetic resonance imaging system 402 comprises a magnet 404. The magnet 404 is a superconducting cylindrical type magnet with a bore 406 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. The magnetic resonance data that is acquired typically acquired for the field of view.

Within the bore 406 of the magnet there is also a set of magnetic field gradient coils 410 which is used for acquisition of preliminary magnetic resonance data to spatially encode magnetic spins within the imaging zone 408 of the magnet 404. The magnetic field gradient coils 410 connected to a magnetic field gradient coil power supply 412. The magnetic field gradient coils 410 are intended to be representative. Typically magnetic field gradient coils 410 contain three separate sets of coils for spatially encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio-frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio-frequency coil 414 is connected to a radio frequency transceiver 416. The radio-frequency coil 414 and radio frequency transceiver 416 may be replaced by separate transmit and receive coils and a separate transmitter and receiver. It is understood that the radio-frequency coil 414 and the radio frequency transceiver 416 are representative. The radio-frequency coil 414 is intended to also represent a dedicated transmit antenna and a dedicated receive antenna. Likewise the transceiver 416 may also represent a separate transmitter and receivers. The radio-frequency coil 414 may also have multiple receive/transmit elements and the radio frequency transceiver 416 may have multiple receive/transmit channels. For example if a parallel imaging technique such as SENSE is performed, the radio-frequency could 414 will have multiple coil elements.

The transceiver 416 and the gradient controller 412 are shown as being connected to the hardware interface 404 of the computer system 402.

The memory 110 is further shown as containing pulse sequence commands 330. The pulse sequence commands could for example contain a label which may be compared to the subject pose label 142. This may be used as a quality control check. In this example the pulse sequence commands 430 may be considered to be a protocol.

In this example the measured tomographic data is now k-space data 124'. The memory 110 is further shown as containing pulse sequence commands 430. The pulse sequence commands are an example of the medical imaging system control commands.

The image quality indicator 126 can be used to determine if the k-space data 124' is transferred to the remote processing system 302. The remote processing system 302 is also shown as comprising a computer 304 as well as a hardware interface 104', a processor 106, a user interface 108', and a memory 110'. The memory 110' is further shown as containing machine-executable instructions 450. The memory 110' is further shown as containing the tomographic data database 306. The memory 110' is further shown as containing the k-space data 124' that has been transferred from the computer 102. The k-space data 124' may for example be stored or retrieved from the tomographic data database 306. The machine-executable instructions 450 enable the processor 106' to reconstruct the tomographic image 308 from the k-space data 124'.

The memory 110 is further shown as optionally containing an operator instruction database 422. The operator instruction database 422 contains instructions which may be used to provide to the operator for improving the acquisition of the tomographic data when it is reacquired. The memory 110 is further shown as containing operator instructions 424 that have been retrieved from the operator instruction database 422 in response to the image quality indicator 126 not being sufficient and retriggering the reacquisition of the measured tomographic data 124'.

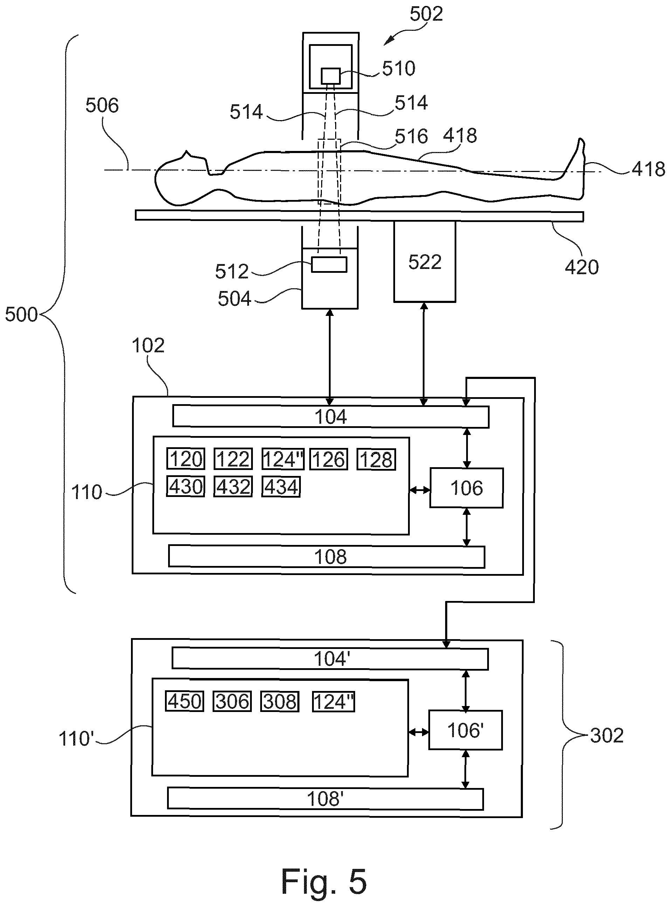

Fig. 5 illustrates a further example of a medical instrument 500. The example in Fig. 5 is similar to that in Fig. 4 except the magnetic resonance imaging system 402 of Fig. 4 has been replaced with a CT or computed tomography system 502 in Fig. 5.

The CT system 502 comprises a rotating gantry 504. The gantry 504 rotates about an axis of rotation 506. There is a subject 418 reposing on a subject support 420. Within the gantry 504 is the X-ray tube 510.

The subject support 420 is shown as being supported by an optional subject support actuator 522. For example, the subject 418 can be brought into the image zone 516. The subject support actuator 522 can hold the subject 418 there until the image quality indicator 126 meets a predetermined criterion and enables it to forward the tomographic data to the remote processing system 302.

The X-ray tube 510 produces X-rays 514 that pass through the subject 418 and are received by a detector 512. Within the area of the box 516 is an imaging zone where CT or computer tomography images of the subject 418 can be made. The CT system 502 is shown as being controlled by computer system 102. The hardware interface 104 allows the processor 106 to exchange messages and control the CT system 502.

In Fig. 5 the measured tomographic data is X-ray attenuation profiles 124". As with Fig. 5, the X-ray attenuation profiles 124" may be forwarded to the remote processing system 302. The functioning of the tomographic data database 306 is analogous to that of Fig. 4. The computer 304 may then reconstruct the X-ray attenuation profiles 124" into the tomographic image 308.

The remote processing system 302 in Figs. 4 and 5 may sometimes be part of the medical instrument 400 or 500.

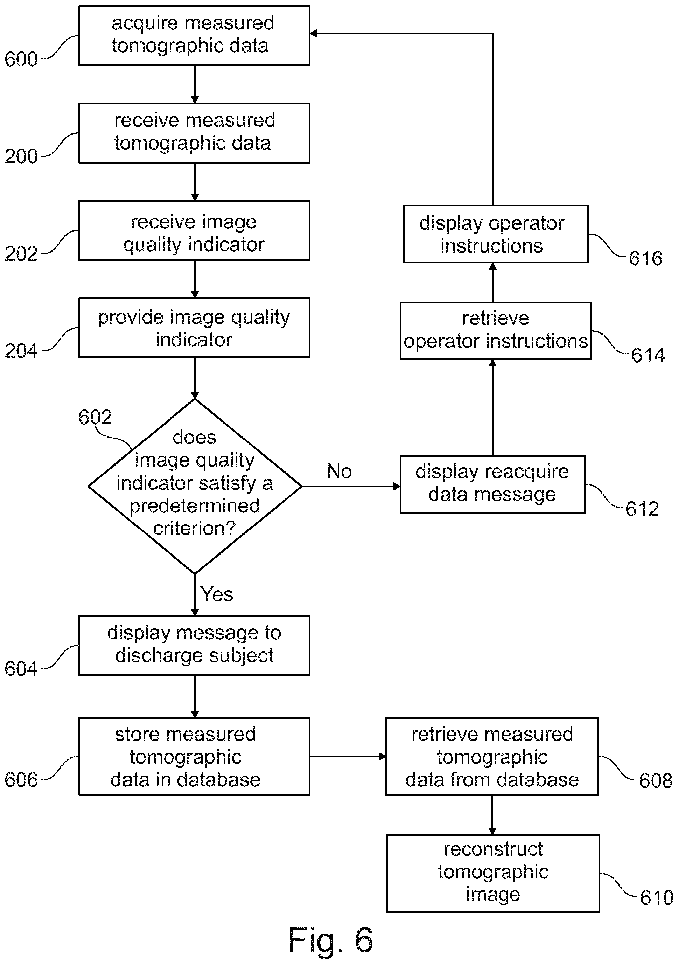

Fig. 6 shows a flowchart which illustrates a method of operating the medical instruments illustrated in Figs. 3, 4 or 5. First in step 600 the medical instrument is controlled to acquire the measured tomographic data 124, 124' or 124". Next steps 200-204 are performed as is illustrated in Fig. 2. After step 204 step 206 is performed; this is a decision box. The question is "does the imaging quality indicator satisfy a predetermined criterion?" If the answer is yes then the method proceeds to box 604. In box 604 a message is displayed informing the operator to discharge the subject. Step 604 may be optional. After step 604 step 606 is performed. In step 606 the measured tomographic data 124, 124', 124" is stored in the tomographic data database 306. After step 606 step 608 is performed. In this step the tomographic data 124, 124', 124" is retrieved from the tomographic data database 306. After step 608 in step 610 the tomographic image 308 is reconstructed from the measured tomographic data 124, 124', 124".

Returning to decision box 602, if the image quality indicator 126 does not satisfy the predetermined criterion then the method proceeds to step 612. Step 612 is optional. In step 612 the measured tomographic data message is displayed to the operator. This informs the operator that the data from the acquisition should be reacquired. Step 614 and step 616 are also optional. In step 614 operator instructions 434 are retrieved from the instruction database 432 in response to the image quality indicator 126 not meeting a predetermined criterion. This information may help the operator to reacquired the data with a higher quality. After steps 612, 614 and 616 are performed the method returns to step 600 and the system reacquires the measured tomographic data 124, 124', 124". If steps 612, 614 or 616 are not performed the method proceeds directly from step 602 to step 600.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

100 medical instrument
102 computer
104 hardware interface
104' hardware interface
106 processor
106' processor
108 user interface (operator signaling system)
110 memory
110' memory
120 machine executable instructions
122 tomographic data assessment module
124 measured tomographic data
124' k-space data
124" X-ray attenuation profiles
126 image quality indicator
126' sufficent image quality indicator
126" insufficent image quality indicator
128 rendering of image quality indicator
200 receive measured tomographic data
202 receive an image quality indicator by inputting the measured tomographic data into the tomographic data assessment module response to inputting the measured tomographic data
204 provide the image quality indicator to an operator using an operator signaling system
300 medical instrument
302 remote processing system
304 computer
306 tomographic data database
308 tomographic image
310 medical imaging system
312 display reacquire data message
314 display discharge subject message
400 medical instrument
402 magnetic resonance imaging system
404 magnet
406 bore of magnet
408 imaging zone
409 field of view
410 magnetic field gradient coils
412 magnetic field gradient coil power supply
414 radio-frequency coil
416 transceiver
418 subject
420 subject support
422 operator instruction database
424 operator instructions
430 pulse sequence commands (example of medical imaging system control commands)
432 instruction database
434 operator instructions
450 machine executable instructions
500 medical instrument
502 CT system
504 gantry
506 axis of rotation
510 X-ray tube
512 detector
514 X-rays
516 imaging zone
520 subject support acutator
530 CT system control commands (example of medical imaging system control commands)
600 acquire the measured tomographic data by controlling the medical imaging system with the medical imaging system control commands
602 does the image quality indicator satisfy a predetermined criterion
604 display a discharge subject message to the operator if the image quality indicator does not satisfy the predetermined criterion
606 store the measured tomographic data in a tomographic data database system of a remote processing system
608 retrieve the measured tomographic data from the tomographic data database
610 reconstruct the tomographic image from the measured tomographic data
612 display a reacquire data message to the operator if the image quality indicator does not satisfy the predetermined criterion
614 retrieve the operator instructions from the instruction database if the reacquire data message is displayed
616 display the operator instructions on the display

## Claims

1. A medical instrument (100, 300, 400, 500) comprising:
- a memory (110) storing machine executable instructions (120) and a tomographic data assessment module (122); and
- a processor (106) configured for controlling the medical instrument, wherein execution of the machine executable instructions causes the processor to:
receive (200) measured tomographic data (124, 124', 124"), wherein the measured tomographic data is configured for being reconstructed into a tomographic image (308) of a subject (418);
receive (202) an image quality indicator (126, 126', 126") by inputting the measured tomographic data into the tomographic data assessment module, wherein the tomographic data assessment module is configured for generating the image quality indicator in response to inputting the measured tomographic data; and
provide (204) the image quality indicator using an operator signaling system (108).

2. The medical instrument of claim 1, wherein the medical instrument further comprises a medical imaging system (310, 402, 502) configured for acquiring the measured tomographic data from an imaging zone (408, 516), wherein the memory further comprises medical imaging system control commands (430, 530) configured for controlling the medical imaging system to acquire the measured tomographic data, wherein execution of the machine executable instructions further causes the processor to acquire (600) the measured tomographic data by controlling the medical imaging system with the medical imaging system control commands.

3. The medical instrument of claim 2, wherein the medical imaging system further comprises a subject support (420, 520) configured for moving at least a portion of the subject within the imaging zone, wherein execution of the machine executable instructions further causes the processor to:
- control the subject support to move the at least a portion of the subject within the imaging zone before controlling the medical imaging system to acquire the measured tomographic data; and
- provide the image quality indicator to the operator using an operator signaling system while the subject is still supported at least partially within the imaging zone.

4. The medical instrument of claim 2 or 3, wherein the medical imaging system is a magnetic resonance imaging system (402), wherein the medical imaging system control commands are pulse sequence commands (430), and wherein the measured tomographic data is k-space data (124').

5. The medical instrument of claim 4, wherein any one of the following:
- wherein the pulse sequence commands are according to a compressed sensing magnetic resonance imaging protocol configured for acquiring the measured tomographic data from multiple magnetic resonance imaging antennas, wherein the tomographic data assessment module is configured for at least partially providing the image quality indicator using magnetic resonance data from a single magnetic resonance antenna selected from the multiple magnetic resonance imaging antennas;
- wherein the pulse sequence commands are according to a self-navigating magnetic resonance imaging protocol that embeds self-navigator data within the k-space data, wherein the tomographic data assessment module is configured for at least partially providing the image quality indicator using the self-navigator data; and
- combinations thereof.

6. The medical instrument of any one of claims 1 through 3, wherein the medical imaging system is a computed tomography imaging system (502), and wherein the measured tomographic data comprises measured X-ray attenuation profiles (124").

7. The medical instrument of any one of the preceding claims, wherein the tomographic data assessment module is configured for accelerating the generation of the image quality indicator using any one of the following:
- subsampling the measured tomographic data;
- reconstructing a low-resolution image from the measured tomographic data, wherein the low-resolution image has a lower resolution than the tomographic image; and
- reconstructing a single slice of the tomographic image from the measured tomographic data.

8. The medical instrument of any one of the preceding claims, wherein the measured tomographic data comprises redundant data, wherein the tomographic data assessment module is configured to at least partially generate the image quality indicator using the redundant data.

9. The medical instrument of any one of the preceding claims, wherein the tomographic data assessment module is implemented as any one of the following:
- a neural network trained to receive as input the measured tomographic data and in response output the image quality indicator;
- a predetermined logic module configured to receive as input the measured tomographic data and in response output the image quality indicator;
- an operator-controlled module configured to generated and display intermediate images or values for approval by the operator; and
- combinations thereof.

10. The medical instrument of any one of the preceding claims, wherein execution of the machine executable instructions further causes the processor to store (606) the measured tomographic data in a tomographic data database system (306) of a remote processing system (302) if (602) the image quality indicator satisfies a predetermined criterion, and wherein the remote processing system is configured to:
- retrieve (608) the measured tomographic data from the tomographic data database; and
- reconstruct (612) the tomographic image from the measured tomographic data.

11. The medical instrument of any one of the preceding claims, wherein the operator signaling system further comprises a computer display configured for displaying the image quality indicator, wherein execution of the machine executable instructions further causes the processor to:
- display (612) a reacquire data message to the operator if the image quality indicator does not satisfy the predetermined criterion; and/or
- display (604) a discharge subject message to the operator if the image quality indicator does not satisfy the predetermined criterion.

12. The medical instrument of claim 11, wherein the memory further comprises an instruction database (422) comprising operator instructions (424) that describe how to improve measured tomographic data quality, wherein execution of the machine executable instructions further causes the processor to:
- retrieve (614) the operator instructions from the instruction database if the reacquire data message is displayed; and
- display (616) the operator instructions on the display.

13. The medical instrument of any one of the preceding claims, wherein the image quality indicator is any one of the following:
- a binary indicator (126', 126") indicating a sufficient image quality and an insufficient image quality;
- a numerical indicator;
- a lower contrast and/or lower resolution image than the tomographic image.
- an operator provided assessment; and
- combinations thereof.

14. A method of operating a medical instrument (100, 300, 400, 500), wherein the method comprises:
- receiving (200) measured tomographic data (124, 124', 124"), wherein the measured tomographic data is configured for being reconstructed into a tomographic image (308) of a subject (418);
- receiving (202) an image quality indicator by inputting the measured tomographic data into a tomographic data assessment module (122), wherein the tomographic data assessment module is configured for generating an image quality indicator (126, 126', 126") in response to inputting the measured tomographic data; and
- providing (204) the image quality indicator to an operator using an operator signaling system (108).

15. A computer program product comprising machine executable instructions (120) and a tomographic data assessment module (122), wherein execution of the machine executable instructions causes the processor to:
- receive (200) measured tomographic data (124, 124', 124"), wherein the measured tomographic data is configured for being reconstructed into a tomographic image (308) of a subject (418);
- receive (202) an image quality indicator (126, 126', 126") by inputting the measured tomographic data into the tomographic data assessment module, wherein the tomographic data assessment module is configured for generating the image quality indicator in response to inputting the measured tomographic data; and
- provide (204) the image quality indicator using an operator signaling system (108).
